# EUROPEAN PATENT SPECIFICATION

(11) **EP 3 600 238 B1**
(45) Date of publication and mention of the grant of the patent: **05.06.2024**
(21) Application number: 18791029.4
(22) Date of filing: 24.04.2018
(51) Int. Cl.: A61Q 11/00, A61K 8/64, A61K 8/97, A61K 36/18, A61K 38/01, A61K 8/41, A61K 8/9794, A61K 36/899

(54) **ORAL CARE PRODUCTS FOR TREATING WHITE SPOT LESIONS**
MUNDPFLEGEPRODUKTE ZUR BEHANDLUNG VON WEISSFLECKIGEN LÄSIONEN
PRODUITS DE SOIN BUCCAL POUR LE TRAITEMENT DE LÉSIONS DE TACHES BLANCHES

(30) Priority: 24.04.2017 US 201762489361 P
(43) Date of publication of application: 05.02.2020
(73) Proprietor: BASF SE, 67056 Ludwigshafen (DE)
(72) Inventor: KILPATRICK-LIVERMAN, LaTonya, New York, New York 10022 (US); QIU, Jianhong, New York, New York 10022 (US); SUBKOWSKI, Thomas, 67054 Ludwigshafen (DE); JENEWEIN, Stefan, 67054 Ludwigshafen (DE); KAROS, Marvin, 67054 Ludwigshafen (DE); BOLLSCHWEILER, Claus, 67054 Ludwigshafen (DE); SCHNEIDER, Nina, 67054 Ludwigshafen (DE); SANTARPIA, Ralph, New York, New York 10022 (US); WENDEL, Volker, 67054 Ludwigshafen (DE)
(74) Representative: BASF IP Association
(86) International application number: PCT/US2018/029121
(87) International publication number: WO 2018/200508

(56) References cited:
- WO-A1-2017/072103
- WO-A1-2017/072104
- WO-A1-2017/072105
- WO-A1-2017/074964
- US-A1- 2006 222 602
- US-A1- 2007 154 411
- US-A1- 2009 196 837
- US-A1- 2011 097 448

## Description

Dental enamel is a thin, hard layer of calcified material that covers the crown of teeth. The major mineral component of dental enamel is hydroxyapatite, a crystalline form of calcium phosphate. Poorly mineralized regions beneath, within or adjacent to intact enamel often appear as white spots on the teeth because such poorly mineralized regions are porous and reflects light differently from sound enamel. In most cases, white spots are the result of demineralization, for example from acids produced by cariogenic bacteria, which break down the hydroxyapatite into calcium and phosphate ions, leading to porous regions within the enamel. Other common causes of poor mineralization and associated white spot lesions include xerostomia, trauma, and arrested decay that has only partially remineralized around fixed orthodontic appliances, which may provide shelter for bacteria or interfere with normal remineralization. Conditions that interfere with tooth development, such as fluorosis, hypomineralization/hypomaturation, mineral deficiencies, and hypoplasia, can also cause white spot lesions.

In the past, white spots were often treated invasively, as incipient cavities. But not all white spots are the result of active, irreversible decay, and minimally invasive treatments are preferable where feasible. Pastes, creams, and topical treatments, typically providing fluoride therapy or some form of calcium phosphate paste, may provide some degree of remineralization, but may not address the cosmetic effects of visible white spots US2006/222602A1 discloses a process for remineralizing dental lesions in teeth with a composite material comprising calcium salts and protein components selected from proteins, protein hydrolysates and protein hydrolysate derivatives, e.g. from plant protein sources, with preferred Mw 600-4000D. Whitening the teeth may make white spots less noticeable by reducing the contrast with the surrounding enamel, but this does not address the underlying poor mineralization and does not entirely eliminate the color contrast differences. More invasive approaches to dealing with white spot lesions include microabrasion, acid etching, composite restorations, and veneers or crowns. Where possible, however, it is desirable to maintain the integrity of the tooth structure.

There is a need for improved oral care compositions to provide alternative noninvasive treatments for white spot lesions on the teeth.

### BRIEF SUMMARY

The present inventors have unexpectedly found that partially hydrolyzed wheat protein is effective in treating, repairing, mitigating, and/or reducing the incidence, size or visibility of white spot lesions on teeth.

The partially hydrolyzed wheat proteins for use in treating, repairing, mitigating, and/or reducing the incidence of white spot lesions on teeth comprise oligo- and polypeptide molecules having a molecular weight distribution of about 500D to about 15000 D, e.g. 1000D to 5000 D, e.g., wherein at least 90% of the oligo- and polypeptide molecules are from about 500 to about 15000 D, e.g., as measured by gel permeation chromatography (GPC). They are prepared for formulation with ingredients of a suitable orally acceptable carrier, by diluting in buffer, e.g., a phospate buffer such as Na2HPO4 buffer (1.5 mM) and CaCl₂ (2.5 mM), to provide a buffered solution having a desired pH, e.g. pH 6-8, e.g., pH 7-8, e.g., about pH 7.5, filtering and centrifuging the solution to obtain a filtrate comprising the partially hydrolyzed plant protein. A biocide (for example cetylpyridinium chloride, e.g., at 0.1%) and/or fluoride may be added to the filtrate. The partially hydrolyzed plant protein may then be combined with components of an orally acceptable carrier, for example a toothpaste, or oral gel or mouthwash base, to provide an oral care composition for use in treating, repairing, mitigating, and/or reducing the incidence, size or visibility of white spot lesions on teeth.

The invention thus relates to an oral care composition for use in the treatment as set out in the claims (for treating, repairing, mitigating, and/or reducing the incidence, size or visibility of white spot lesions on teeth of a patient in need thereof), comprising applying to the teeth an effective amount of an oral care composition, for example a dentifrice, comprising:
a) a) partially hydrolyzed wheat protein, comprising oligo- and polypeptide molecules having a molecular weight wherein at least 90% of the oligo- and polypeptide molecules are from about 500 to about 15000 D (e.g., as measured by GPC); e.g., at least 95% of the oligo- and polypeptide molecules are from about 500 to about 10000 D, e.g. about 1000 to about 5000 D; e.g. having a molecular weight distribution as follows: about 65% < 3500D, about 84-92% < 6000D, about 0.9-3.6% > 9000D, and about 0.1-0.6% > 13000D;
b) an orally acceptable carrier.

In particular the present invention provides the use of partially hydrolyzed plant protein in treating, repairing, mitigating, and/or reducing the incidence, size or visibility of white spot lesions on teeth, wherein partially hydrolyzed plant protein comprises oligo- and polypeptide molecules wherein at least 90% of the oligo- and polypeptide molecules are from about 500 to about 15000 D (e.g., as measured by GPC); e.g. wherein at least 95% of the plant protein is from about 500 to about 10000 D, e.g. about 1000 to about 5000 D; e.g. having a molecular weight distribution as follows: about 65% < 3500D, about 84-92% < 6000D, about 0.9-3.6% > 9000D, and about 0.1-0.6% > 13000D, e.g., for use in any of Methods 1, *et seq.* or IA, *et seq.* wherein the oral care composition is applied at least once a day, e.g., 1-3 times per day, for a period of at least a week, e.g. at least 10 days, e.g., for two weeks to six months, e.g., until the white spot lesions are reduced or no longer visible.

The present description discloses an oral care composition for use as claimed which is made by
a) diluting a solution of a partially hydrolyzed plant protein, comprising oligo- and polypeptide molecules having a molecular weight distribution of about 500 to about 10000 D, e.g. about 1000 to about 5000 D, with an aqueous buffer solution, e.g., a phosphate buffer solution, e.g., to obtain a solution having the desired pH, e.g., pH 6-8, e.g., pH 7-8;
b) filtering and centrifuging the solution product of a) to obtain a filtrate comprising the partially hydrolyzed plant protein;
c) adding fluoride (e.g., in the form of an orally acceptable salt containing fluoride, for example sodium fluoride or sodium monofluorophosphate), and optionally a biocide (e.g., cetylpyridinium chloride at an effective amount, e.g., 0.01 to 1%, e.g., about 0.1% by weight of the filtrate) to the filtrate product of b);
d) admixing the product of c) to components of an orally acceptable carrier to obtain an oral care composition comprising the partially hydrolyzed plant protein in an amount of from 0.01 weight % to 3 weight % by total weight of the composition.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION

The following description of the preferred embodiment(s) is merely exemplary in nature and is in no way intended to limit the invention, its application, or uses.

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

### Hydrolyzed plant proteins

Hydrolyzed wheat protein is typically obtained by enzymatically hydrolyzing wheat proteins using endoproteases and exoproteases. Hydrolyzed wheat protein may also be obtained through acid or alkaline hydrolysis. Methods of preparing hydrolyzed wheat protein would be known to the person skilled in the art of protein chemistry. However, hydrolyzed wheat protein is also commercially available as Gluadin^{®} W20, Gluadin W40 from BASF, and as Wheatpro^{®} from IKEDA. Gluadin W20 is a partial hydrolysate obtained through enzymatic hydrolysis of wheat gluten. It contains at least 20.0 % of dry substance. Gluadin W40 is a partial hydrolysate obtained through enzymatic hydrolysis of wheat gluten. It contains at least 40.0 % of dry substance.

In another embodiment, the hydrolyzed plant protein is made from processed wheat protein which is free of gluten.

Hydrolyzed rice protein is typically obtained by enzymatically hydrolyzing rice protein using endoproteases and exoproteases. Hydrolyzed rice protein may also be obtained through acid or alkaline hydrolysis. Methods of preparing hydrolyzed rice protein would be known to the person skilled in the art of protein chemistry. However, hydrolyzed rice protein is also commercially available as Gluadin^{®} R from BASF, and as Rice Pro-Tein BK-S^{®} from TRI-K Industries

The hydrolyzed plant protein for use in accordance with the present disclosure is not *fully* hydrolyzed and thus is sometimes referred to as "partially hydrolyzed" to emphasize this point. By "partially hydrolyzed" it is meant that at least some, but not all, of the peptide bonds are hydrolyzed. Thus, the hydrolyzed plant protein typically comprises a mixture of amino acids and peptides of varying size. MW of relevant active ingredient is in the range of about 500 - about 10,000 D, for example about 1000 to about 5000 D.

In some embodiments, the hydrolyzed plant protein for use in accordance with the present disclosure is present in the composition in an amount of from 0.01 weight % to 3 weight % by total weight of the composition. In some embodiments, the hydrolyzed plant protein is present in the composition in an amount of from 0.1 weight % to 3 weight %, or from 0.1 weight % to 2 weight %, or from 0.1 weight % to 1 weight % by total weight of the composition. In other embodiments, the hydrolyzed plant protein is present in the composition in an amount of from 0.05 weight % to 1 weight %, or from 0.1 weight % to 0.5 by total weight of the composition In further embodiments, the hydrolyzed plant protein is present in the composition in an amount of from 0.5 weight % to 3 weight %, or from 0.5 weight % to 2 weight %, or from 0.5 weight % to 1 weight % by total weight of the composition. In still further embodiments, the hydrolyzed plant protein is present in the composition in an amount of from 1 weight % to 3 weight %, or from 1 weight % to 2 weight % by total weight of the composition.

In one embodiment, the compositions for use in accordance with the present disclosure comprise both hydrolyzed wheat protein and hydrolyzed rice protein. In this embodiment, the hydrolyzed wheat protein and the hydrolyzed rice protein may be present in the composition in the amounts defined above. Optionally, the total amount of hydrolyzed wheat protein and hydrolyzed rice protein in the composition is from 0.1 weight % to 3 weight %, or from 0.1 weight % to 2 weight %, or from 0.1 weight % to 1 weight %, or from 0.1 weight % to 0.5 weight % by total weight of the composition. In some embodiments, the total amount of hydrolyzed wheat protein and hydrolyzed rice protein in the composition is from 1 weight % to 3 weight %, or from 1 weight % to 2 weight %, by total weight of the composition.

### Orally acceptable carrier and optional ingredients

The expression "orally acceptable carrier" as used herein denotes a carrier made from materials that are safe and acceptable for oral use in the amounts and concentrations intended, for example materials as would be found in conventional toothpaste and mouthwash. Such materials include water or other solvents that may contain a humectant such as glycerin, sorbitol, xylitol and the like. In some aspects, the term "orally acceptable carrier" encompasses all of the components of the oral care composition except for the hydrolyzed plant protein and the fluoride. In other aspects, the term refers to inert or inactive ingredients that serve to deliver the hydrolyzed plant protein, and/or any other functional ingredients, to the oral cavity.

Orally acceptable carriers for use in accordance with the present disclosure include conventional and known carriers used in making mouth rinses or mouthwashes, toothpastes, tooth gels, tooth powder, lozenges, chewing gums, beads, edible strips, tablets and the like. Carriers should be selected for compatibility with each other and with other ingredients of the composition.

The following non-limiting examples are provided. In a toothpaste composition, the carrier is typically a water/humectant system that provides a major fraction by weight of the composition. Alternatively, the carrier component of a toothpaste composition may comprise water, one or more humectants, and other functional components other than the hydrolyzed wheat protein or hydrolyzed rice protein. In a mouth rinse or a mouthwash formulation, the carrier is typically a water/alcohol liquid mixture in which the hydrolyzed wheat protein or hydrolyzed rice protein is dissolved or dispersed. In a dissolvable lozenge, the carrier typically comprises a solid matrix material that dissolves slowly in the oral cavity. In chewing gums, the carrier typically comprises a gum base, while in an edible strip, the carrier typically comprises one or more film forming polymers.

The oral care compositions for use in accordance with the present disclosure may further comprise one or more additional ingredients selected from abrasives, pH modifying agents, surfactants, foam modulators, thickening agents, viscosity modifiers, humectants, anti-calculus or tartar control agents, sweeteners, flavorants, colorants and preservatives. These ingredients may also be regarded as carrier materials. Non-limiting examples are provided below.

In one embodiment a composition for use in accordance with the present disclosure comprises at least one abrasive, useful, for example, as a polishing agent. Any orally acceptable abrasive can be used, but the type, fineness (particle size) and amount of abrasive should be selected so that tooth enamel is not excessively abraded during normal use of the composition. Suitable abrasives include, without limitation, silica, for example in the form of silica gel, hydrated silica or precipitated silica, alumina, insoluble phosphates, calcium carbonate, resinous abrasives such as urea-formaldehyde condensation products and the like. Among insoluble phosphates useful as abrasives are orthophosphates, polymetaphosphates and pyrophosphates. Illustrative examples are dicalcium orthophosphate dihydrate, calcium pyrophosphate, [beta]-calcium pyrophosphate, tricalcium phosphate, calcium polymetaphosphate and insoluble sodium polymetaphosphate. One or more abrasives are optionally present in the oral care compositions of the present invention in an amount of 1 weight % to 5 weight % by total weight of the composition. The average particle size of an abrasive, if present, is generally 0.1 to 30µm, and preferably, 5 to 15µm.

In a further embodiment an oral care composition for use in accordance with the present disclosure comprises at least one bicarbonate salt, useful, for example, to impart a "clean feel" to teeth and gums due to effervescence and release of carbon dioxide. Any orally acceptable bicarbonate can be used, including, without limitation, alkali metal bicarbonates such as sodium and potassium bicarbonates, ammonium bicarbonate and the like. One or more bicarbonate salts are optionally present in a total amount of 1 weight % to 10% by weight of the composition.

In a still further embodiment, an oral care composition for use in accordance with the present disclosure comprises at least one pH modifying agent. Such agents include acidifying agents to lower pH, basifying agents to raise pH and buffering agents to control pH within a desired range. For example, one or more compounds selected from acidifying, basifying and buffering agents can be included to provide a pH of 2 to 10, or in various illustrative embodiments a pH of 2 to 8, 3 to 9, 4 to 8, 5 to 7, 6 to 10, or 7 to 9. Any orally acceptable pH modifying agent can be used, including, without limitation, carboxylic, phosphoric and sulfonic acids, acid salts (for example, monosodium citrate, disodium citrate, monosodium malate), alkali metal hydroxides such as sodium hydroxide, carbonates such as sodium carbonate, bicarbonates, borates, silicates, phosphates (for example, monosodium phosphate, trisodium phosphate, pyrophosphate salts) imidazole and the like. One or more pH modifying agents are optionally present in a total amount effective to maintain the composition in an orally acceptable pH range.

In a still further embodiment an oral care composition for use in accordance with the present disclosure comprises at least one surfactant, useful, for example, to provide enhanced stability to the composition and the components contained therein, to aid in cleaning a dental surface through detergent action, and to provide foam upon agitation (for example, during brushing with a dentifrice composition of the invention). Any orally acceptable surfactant, including those which are anionic, nonionic or amphoteric, can be used. Suitable anionic surfactants include, without limitation, watersoluble salts of C₈₋₂₀ alkyl sulfates, sulfonated monoglycerides of C₈₋₂₀ fatty acids, sarcosinates, taurates and the like. Suitable nonionic surfactants include, without limitation, poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants , without limitation, derivatives of C₈₋₂₀ aliphatic secondary and tertiary amines having an anionic group such as carboxylate, sulfate, sulfonate, phosphate or phosphonate. A suitable example is cocoamidopropyl betaine. One or more surfactants are optionally present in a total amount of 0.01 weight % to 10 weight %, for example, from 0.05 weight % to 5 weight % or from 0.1 weight % to 2 weight % by total weight of the composition.

In a still further embodiment, an oral care composition for use in accordance with the present disclosure comprises at least one foam modulator, useful, for example, to increase the amount, thickness or stability of foam generated by the composition upon agitation. Any orally acceptable foam modulator can be used including, without limitation, polyethylene glycols (PEGs). One or more PEGs are optionally present in a total amount of from 0.1 weight % to 10 weight by total weight of the composition.

In a still further embodiment, an oral care composition for use in accordance with the present disclosure comprises at least one thickening agent, useful, for example, to impart a desired consistency and/or mouth feel to the composition. Any orally acceptable thickening agent can be used including, without limitation, carbomers (carboxyvinyl polymers), carrageenans, cellulosic polymers such as hydroxyethylcellulose, carboxymethylcellulose (CMC) and salts thereof, natural gums such as karaya, xanthan, gum arabic and tragacanth, colloidal magnesium aluminum silicate, colloidal silica and the like. One or more thickening agents are optionally present in a total amount of 0.01 weight % to 15 weight %, by total weight of the composition.

In a still further embodiment a composition for use in accordance with the present disclosure comprises at least one viscosity modifier, useful, for example, to inhibit settling or separation of ingredients or to promote re-dispersion of ingredients upon agitation of a liquid composition. Any orally acceptable viscosity modifier can be used including, without limitation, mineral oil, petrolatum, clays, silica and the like. One or more viscosity modifiers are optionally present in a total amount of 0.01 weight % to 10 weight %, by total weight of the composition.

In a still further embodiment, an oral care composition for use in accordance with the present disclosure at least one humectant which may be used to prevent hardening of a toothpaste upon exposure to air, or in the case of a mouthwash, to provide improved moisturizing and mouthfeel and enhance the miscibility of poorly soluble components such a flavoring oils. Any orally acceptable humectant can be used, including, without limitation, polyhydric alcohols such as glycerin, sorbitol, xylitol or low molecular weight PEGs. Most humectants also function as sweeteners. One or more humectants are optionally present in a total amount of 1 weight % to 50 weight % by total weight of the composition.

In a still further embodiment, an oral care composition for use in accordance with the present disclosure comprises at least one sweetener which enhances taste of the composition. Any orally acceptable natural or artificial sweetener can be used including, without limitation, dextrose, sucrose, maltose, dextrin, mannose, xylose, ribose, fructose, levulose, galactose, corn syrup, partially hydrolyzed starch, hydrogenated starch hydrolysate, sorbitol, mannitol, xylitol, maltitol, isomalt, aspartame, neotame, saccharin and salts thereof, dipeptide-based intense sweeteners, cyclamates and the like. One or more sweeteners are optionally present in a total amount of 0.005 weight % to 5 weight % by total weight of the composition.

In a still further embodiment, an oral care composition for use in accordance with the present disclosure comprises at least one flavorant which enhances the taste of the composition. Any orally acceptable natural or synthetic flavorant can be used including, without limitation, vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences, and the like. Also encompassed within flavorants are ingredients that provide fragrance and/or other sensory effects in the mouth, including cooling or warming effects. Such ingredients illustratively include menthol, menthyl acetate, menthyl lactate, camphor, eucalyptus oil, eucalyptol, eugenol, cassia, oxanone, α-irisone, thymol, linalool, benzaldehyde, cinnamaldehyde, N-ethyl-p-menthan-3-carboxamine, N,2,3-trimethyl-2-isopropylbutanamide, 3 -( 1 -menthoxy)-propane- 1 ,2-diol, cinnamaldehyde glycerol acetal (CGA), menthone glycerol acetal (MGA) and the like. One or more flavorants are optionally present in a total amount of 0.01 weight % to 5 weight %, by total weight of the composition.

In a still further embodiment, an oral care composition for use in accordance with the present disclosure comprises at least one colorant. A colorant can serve a number of functions. These include providing a white or light-colored coating on a dental surface, indicating locations on a dental surface that have been effectively contacted by the composition, and/or modifying the appearance of the composition to enhance attractiveness to the consumer. Any orally acceptable colorant can be used including, without limitation, talc, mica, magnesium carbonate, calcium carbonate, magnesium silicate, magnesium aluminum silicate, silica, titanium dioxide, zinc oxide, iron oxide, ferric ammonium ferrocyanide, manganese violet, titaniated mica, bismuth oxychloride and the like. One or more colorants are optionally present in a total amount of 0.001 weight % to 20 weight % by total weight of the composition.

In a still further embodiment, an oral care composition for use in accordance with the present disclosure comprises a preservative. The preservative may be selected from parabens, potassium sorbate, benzyl alcohol, phenoxyethanol, polyaminopropryl biguanide, caprylic acid, sodium benzoate and cetylpyridinium chloride. In some embodiments, the preservative is present at a concentration of from about 0.001 to about 1 weight %, by total weight of the composition.

The following examples illustrate compositions for use in accordance with the present disclosure. The exemplified compositions are illustrative and do not limit the scope of the disclosure.

### EXAMPLES

### Example 1 - White Spot Reduction

A partially hydrolyzed wheat protein preparation is made using GLUADIN W40 from BASF. The hydrolysate is diluted to the final concentration using DI water. After neutralizing to a pH of 7.5, the solution is filtered and centrifuged. Four liquids are tested to determine the efficacy of this wheat hydrolysate in reducing white spot lesions:
1: 2% wheat hydrolysate water solution, w/ 0.04% CPC as preservative
2: 0.2% wheat hydrolysate water solution, w/ 0.04% CPC as preservative
3: Mouthwash with 0.2% wheat hydrolysate
4. Control mouthwash

The two mouthwash formulations are set forth in Table 1:

**Table 1**

| Ingredients (wt%) | Mouthwash with 0.2% wheat hydrolysate | Control mouthwash |
|---|---|---|
| Demineralized water | 78.975 | 79.175 |
| Poloxamer 338 | 0.5 | 0.5 |
| Sodium Saccharin | 0.02 | 0.02 |
| Wheat Hydrolysate | 0.2 | -- |
| Cetylpyridinium Chloride | 0.04 | 0.04 |
| Sodium Fluoride | 0.05 | 0.05 |
| Potassium Sorbate | 0.05 | 0.05 |
| 99.0% - 101.0% Glycerin | 7.5 | 7.5 |
| Sorbitol (70% solution) | 5.5 | 5.5 |
| Propylene Glycol | 7 | 7 |
| Optacool (coolant flavor, Symrise) | 0.025 | 0.025 |
| L-Menthol | 0.02 | 0.02 |
| Peppermint Flavor | 0.12 | 0.12 |

Artificial saliva is prepared, using MgCl₂ 0.2 mM, CaCl₂·H₂O 1mM, HEPES buffer 20 mM, KH₂PO₄ 4 mM, KCl 16 mM, NH₄Cl 4.5 mM, NaF 300 ppm and 0.05% NaN₃, pH ~ 7.0.

Human extracted teeth with white spot(s) are gently brush cleaned and also screened by dentist. The screened teeth are then stored in deionized water at ca. 4°C until the treatments are started.

The stored teeth are taken out, slightly dried with paper towel before the base line images of the white spots and adjacent sound areas are taken. The contrast values between the white spot and adjacent sound areas are quantified using imaging software.

The teeth are then soaked in one of the above four test solutions for 30 minutes, 15ml for each tooth, at 37°C, while being spun at 100 rpm. The teeth are then rinsed for 5 minutes with deionized water at 400 rpm. The teeth are then paper towel dried, and the deionized water rinse is repeated. The treated teeth are then incubated in artificial saliva overnight at 37° C.

This treatment, washing and incubation is repeated daily for nine more days, for a total of 10 days of treatment. After the 10 days of treatment is completed, the teeth are rinsed twice with deionized water as described.

In order to avoid false contrast readings, e.g., decreased contrast from loosely deposited mineral on tooth, the teeth are brushed after all treatment/incubation. The teeth are each brushed under tap water, continuously and evenly at all angles for two minutes, rinsed under tap water, and placed onto a paper towel to dry.

Final images of the treated teeth, after brushing, are taken after the teeth are slightly dried with the paper towel. The contrast between the white spot area and the adjacent sound enamel is again quantified. Reduction in contrast (negative value) indicates degree of repair of the white spot. The results are set forth in Table 2:

**Table 2**

| **Material** | **Contrast change** |
|---|---|
| 2% wheat hydrolysate water solution, w/ 0.04% CPC as preservative | -21.4 |
| 0.2% wheat hydrolysate water solution, w/ 0.04% CPC as preservative | -18.6 |
| Mouthwash with 0.2% wheat hydrolysate | -19.5 |
| Control mouthwash | 0.76 |

This data shows that the three solutions containing the wheat hydrolysate reduced the white spot lesions, while the mouthwash without wheat hydrolysate had little effect on the white spots.

## Claims

1. An oral care composition for use in the treatment of white spot lesions on teeth of a patient in need thereof, wherein the oral care composition comprises:
a) partially hydrolyzed plant protein, comprising oligo- and polypeptide molecules wherein at least 90% of the oligo- and polypeptide molecules are from about 500 to about 15000 D; and
b) an orally acceptable carrier;
wherein the partially hydrolyzed plant protein is partially hydrolyzed wheat protein, and wherein the oral care composition is applied at least once a day, e.g., 1-3 times per day, for a period of at least a week, e.g., at least 10 days, e.g., for two weeks to six months, e.g., until the white spot lesions are reduced or no longer visible.

2. The oral care composition for use of claim 1 wherein the partially hydrolyzed plant protein has been adjusted to pH 7-8 by using a phosphate buffer.

3. The oral care composition for use of any foregoing claim wherein the partially hydrolyzed plant protein comprises cetylpyridinium chloride (CPC) at an effective concentration, e.g., 0.1% by weight of the filtrate.

4. The oral care composition for use of any foregoing claim wherein the oral care composition further comprises an effective amount of fluoride.

5. The oral care composition for use of any foregoing claim wherein the partially hydrolyzed plant protein is present in the composition in an amount of from 0.1 weight % to 3 weight % by total weight of the composition.

6. The oral care composition for use of any foregoing claim wherein the oral care composition comprises 100 - 1500 ppm fluoride.

7. The oral care composition for use of any foregoing claim wherein the oral care composition comprises cetyl pyridinium chloride.

8. The oral care composition for use of any foregoing claim wherein the oral care composition is a dentifrice, gel, mouthwash, tablet, toothpowder, or a chewing gum, preferably mouthwash.

9. The oral care composition for use of any foregoing claim wherein the oral care composition is a mouthwash which comprises
a. partially hydrolyzed wheat protein in an amount of from 0.05-1% by weight;
b. a fluoride ion source providing 200 to 1000 ppm fluoride and
c. cetylpyridinium chloride in an amount of 0.01%-0.1% by weight.

## Patentansprüche

1. Mundpflegezusammensetzung zur Verwendung bei der Behandlung von White-Spot-Läsionen auf Zähnen eines Patienten mit Bedarf daran, wobei die Mundpflegezusammensetzung umfasst:
a) teilhydrolysiertes Pflanzenprotein, umfassend Oligo- und Polypeptidmoleküle, wobei wenigstens 90 % der Oligo- und Polypeptidmoleküle von etwa 500 bis etwa 15000 D aufweisen; und
b) einen oral annehmbaren Träger;
wobei das teilhydrolysierte Pflanzenprotein teilhydrolysiertes Weizenprotein ist und wobei die Mundpflegezusammensetzung wenigstens einmal täglich, z.B. 1-3-Mal pro Tag, für einen Zeitraum von wenigstens einer Woche, z.B. Wenigstens 10 Tagen, z.B. Für zwei Wochen bis sechs Monate, z.B. Bis die White-Spot-Läsionen verringerrt oder nicht mehr sichtbar sind, angewendet wird.

2. Mundpflegezusammensetzung zur Verwendung gemäß Anspruch 1, wobei das teilhydrolysierte Pflanzenprotein unter Verwendung eines Phosphatpuffers auf pH 7-8 eingestellt worden ist.

3. Mundpflegezusammensetzung zur Verwendung gemäß einem der vorstehenden Ansprüche, wobei das teilhydrolysierte Pflanzenprotein Cetylpyridiniumchlorid (CPC) in einer wirksamen Konzentration, z.B. 0,1 Gew.-% des Filtrats, umfasst.

4. Mundpflegezusammensetzung zur Verwendung gemäß einem der vorstehenden Ansprüche, wobei die Mundpflegezusammensetzung ferner eine wirksame Menge Fluorid umfasst.

5. Mundpflegezusammensetzung zur Verwendung gemäß einem der vorstehenden Ansprüche, wobei das teilhydrolysierte Pflanzenprotein in der zusammensetzung in einer Menge von 0,1 Gew.-% bis 3 Gew.-% bezogen auf das Gesamtgewicht der Zusammensetzung vorhanden ist.

6. Mundpflegezusammensetzung zur Verwendung gemäß einem der vorstehenden Ansprüche, wobei die Mundpflegezusammensetzung 100-1500 ppm Fluorid umfasst.

7. Mundpflegezusammensetzung zur Verwendung gemäß einem der vorstehenden Ansprüche, wobei die Mundpflegezusammensetzung Cetylpyridiniumchlorid umfasst.

8. Mundpflegezusammensetzung zur Verwendung gemäß einem der vorstehenden Ansprüche, wobei die Mundpflegezusammensetzung eine/ein Zahnpasta, Gel, Mundspülung, Tablette, Zahnpulver oder ein Kaugummi, vorzugsweise eine Mundspülung, ist.

9. Mundpflegezusammensetzung zur Verwendung gemäß einem der vorstehenden Ansprüche, wobei die Mundpflegezusammensetzung eine Mundspülung ist, die umfasst:
a. teilhydrolysiertes Weizenprotein in einer Menge von 0,05-1 Gew.-%;
b. eine Fluoridionenquelle, die 200 bis 1000 ppm Fluorid bereitstellt, und
c. Cetylpyridiniumchlorid in einer Menge von 0,01 Gew.-% bis 0,1 Gew.-%.

## Revendications

1. Composition de soin oral pour une utilisation dans le traitement des lésions de taches blanches sur les dents d'un patient qui en a besoin, dans laquelle la composition de soin oral comprend :
a) une protéine végétale partiellement hydrolysée, comprenant des molécules d'oligopeptide et de polypeptide dans laquelle au moins 90 % des molécules d'oligopeptide et de polypeptide sont d'environ 500 à environ 15 000 D ; et
b) un support acceptable oralement ;
dans laquelle la protéine végétale partiellement hydrolysée est une protéine de blé partiellement hydrolysée, et dans lequel la composition de soin oral est appliquée au moins une fois par jour, par exemple 1-3 fois par jour, pendant une période d'au moins une semaine, par exemple, au moins 10 jours, par exemple, pendant deux semaines à six mois, par exemple, jusqu'à ce que les taches blanches soient réduites ou ne soient plus visibles.

2. Composition de soin oral selon la revendication 1, dans laquelle la protéine végétale partiellement hydrolysée a été ajustée à pH 7-8 à l'aide d'un tampon phosphate.

3. Composition de soin oral pour une utilisation selon l'une quelconque des revendications précédentes, dans laquelle la protéine végétale partiellement hydrolysée comprend du chlorure de cétylpyridinium (CPC) à une concentration efficace, par exemple, 0,1 % en poids du filtrat.

4. Composition de soin oral pour une utilisation selon l'une quelconque des revendications précédentes, dans laquelle la composition de soin oral comprend en outre une quantité effective de fluorure.

5. Composition de soin oral pour une utilisation selon l'une quelconque des revendications précédentes, dans laquelle la protéine végétale partiellement hydrolysée est présente dans la composition en une quantité allant de 0,1 % en poids à 3 % en poids par poids total de la composition.

6. Composition de soin oral pour une utilisation selon une quelconque revendication précédente dans laquelle la composition de soin oral comprend 100 - 1 500 ppm de fluorure.

7. Composition de soin oral pour une utilisation selon l'une quelconque des revendications précédentes, dans laquelle la composition de soin oral comprend du chlorure de cétylpyridinium.

8. Composition de soin oral pour une utilisation selon l'une quelconque des revendications précédentes, dans laquelle la composition de soin oral est un dentifrice, un gel, un bain de bouche, un comprimé, une poudre dentaire ou un chewing-gum, de préférence un bain de bouche.

9. Composition de soin oral pour une utilisation selon l'une quelconque des revendications précédentes, dans laquelle la composition de soin oral est un bain de bouche qui comprend
a. une protéine de blé partiellement hydrolysée en une quantité allant de 0,05 à 1 % en poids ;
b. une source d'ions fluorure fournissant 200 à 1 000 ppm de fluorure et
c. du chlorure de cétylpyridinium en une quantité de 0,01 % à 0,1 % en poids.
